# EUROPEAN PATENT SPECIFICATION

(11) **EP 3 785 014 B1**
(45) Date of publication and mention of the grant of the patent: **25.06.2025**
(21) Application number: 19792287.5
(22) Date of filing: 26.04.2019
(51) Int. Cl.: G01N 15/10, G06N 20/00, G01N 15/14, G01N 33/574, G01N 15/00, G06N 3/04, G06N 5/00, G06N 7/00, G06N 20/10, G06N 20/20, G06N 3/12, G06N 3/126, G01N 15/1429, G06N 5/01

(54) **METHODS OF DIAGNOSING DISEASE USING MICROFLOW CYTOMETRY**
VERFAHREN ZUR DIAGNOSE VON KRANKHEITEN MITTELS MIKRODURCHFLUSSZYTOMETRIE
MÉTHODES DE DIAGNOSTIC D'UNE MALADIE PAR CYTOMÉTRIE EN MICROFLUX

(30) Priority: 27.04.2018 CA 3003032
(43) Date of publication of application: 03.03.2021
(73) Proprietor: Nanostics Inc., Edmonton AB T5J 4P6 (CA)
(72) Inventor: LEWIS, John, Edmonton, Alberta T6H 3E2 (CA); PAPROSKI, Robert, Edmonton, Alberta T5A 4R4 (CA); PINK, Desmond, Edmonton, Alberta T5M 0W4 (CA); VASQUEZ, Catalina, Edmonton, Alberta T6R 0V2 (CA)
(74) Representative: Zwicker, Jörk
(86) International application number: PCT/CA2019/050541
(87) International publication number: WO 2019/204940

(56) References cited:
- EP-A2- 1 894 131
- WO-A1-2017/053592
- WO-A1-2018/075825
- US-A1- 2005 095 611
- US-A1- 2007 009 970
- US-A1- 2012 004 130
- US-A1- 2013 095 575
- US-A1- 2016 169 786
- US-B1- 9 934 364
- US-B2- 10 874 610
- US-B2- 8 725 426
- D.PINK, R.PAPROSKI (O523.03, LBO.14, OT.7.05): "Abstract Book: ISEV2017", JOURNAL OF EXTRACELLULAR VESICLES., vol. 6, no. sup1, 15 May 2017 (2017-05-15), pages 41 - 41, XP055588374, DOI: 10.1080/20013078.2017.1310414
- D.PINK., R.PAPROSKI (PS09.5, PS08.04): "ISEV2018 abstract book", JOURNAL OF EXTRACELLULAR VESICLES, vol. 7, no. sup1, 20 April 2018 (2018-04-20), pages 1461450, XP055810867, Retrieved from the Internet <URL:https://www.tandfonline.com/doi/pdf/10.1080/20013078.2018.1461450?needAccess=true> [retrieved on 20211215], DOI: 10.1080/20013078.2018.1461450
- DESMOND PINK ET AL: "Technical validation of a micro-flow cytometry platform for prostate cancer biomarker discover", vol. 77, no. 13, 30 June 2017 (2017-06-30), pages 3799, XP009524001, ISSN: 0008-5472, Retrieved from the Internet <URL:http://cancerres.aacrjournals.org/lookup/doi/10.1158/1538-7445.AM2017-3799> [retrieved on 20170702], DOI: 10.1158/1538-7445.AM2017-3799
- PINK ET AL.: "Technical validation of a micro-flow cytometry platform for prostate cancer biomarker discover", CANCER RESEARCH, vol. 77, no. 13, July 2017 (2017-07-01), pages 3799 - 3799, XP009524001, DOI: 10.1158/1538-7445.AM2017-3799

## Description

### FIELD OF THE INVENTION

Generally, the present invention relates to diagnostic methods and biomarkers tested therein. More specifically, the present invention relates to the use of extracellular vesicles for clinically significant prostate cancer diagnosis and biomarkers for predicting the same.

### BACKGROUND OF THE INVENTION

Extracellular vesicles (EVs) hold great potential for diagnostics and prognostics in a variety of fields such as immunology, neurology, cardiology, and oncology. EVs include exosomes (30-100 nm), microvesicles (50-2,000 nm), apoptotic bodies (500-4,000 nm), and very large oncosomes (1,000-10,000nm). Healthy and diseased cells continuously release EVs which contain many of the mRNA, miRNA, and protein markers from their cells of origin. EVs have been found in nearly all biological fluids including blood, urine, semen, and cerebrospinal fluid, making them promising targets for minimally-invasive diagnostic assays.

Multiple methods exist for EV characterization (Szatenek R et al., Int J Mol Sci 18(6), 2017). Electron microscopy provides the highest resolution images of EVs but lacks high-throughput data acquisition, cannot easily measure many markers simultaneously, and may require time consuming and complicated data analysis since the raw data are images (Harris JR, Arch Biochem Biophys 581: 3-18, 2015). Nanoparticle tracking analysis and tunable resistive pulse sensing allow rapid enumeration and sizing of particles but are not ideal for characterizing EV markers (Gardiner C et al., J Extracell Vesicles 2, 2013; Vogel R et al., Anal Chem 83(9): 3499-35-6, 2011). Microflow cytometry (µFCM), also referred to as nanoscale or high sensitivity flow cytometry allows high-throughput characterization of the optical properties of particles, allowing quantification of particle size, concentration, and marker abundance for millions of EVs in minutes (Szatenek supra). These desirable characteristics make µFCM well-suited for highsensitivity EV-based clinical assays.

µFCM generates large amounts of data which complicates analysis. A typical 10 µL plasma sample that has been diluted 100-fold can generate over 5,000,000 events each in a single minute of analysis with over a dozen optical properties. In other words, a single µL of plasma can have >109 events. Other liquid biopsy types can have similar concentrations. The analysis of EVs by µFCM can examine at least 500 -50,000 fold more sample events compared to Nanoparticle (NTA) or electron microscopy per sample analysis, providing a greater representative analysis of the whole sample. Traditional cell-based flow cytometry analysis typically involves generating bivariate scatter plots and quantifying event concentration within user-defined regions of interest (ROIs) over 4 quadrants since many cells have similar size and are characterized as marker positive or negative. Such methods are too simplistic for µFCM since EVs range in size and hence in marker abundance which necessitates the development of µFCM analysis tools that can rapidly process very large complex data sets.

When generating an EV-based diagnostic/prognostic assay, EVs must not only be characterized within biological samples but also analyzed for their ability to predict clinically meaningful conditions which can improve patient well-being and/or healthcare economics.

D.PINK, R.PAPROSKI (O523.03, LBO.14, OT.7.05): "Abstract Book: ISEV2017",JOURNAL OF EXTRACELLULAR VESICLES., vol. 6, no. sup1, 15 May 2017 (2017-05-15), pages 41-41, XP055588374,DOI: 10.1080/20013078.2017.1310414, also discloses a prior art method of diagnosing a disease signature for a disease in a patient.

### SUMMARY OF THE INVENTION

According to an aspect of the present invention, there is provided a method of diagnosing disease signature for a disease in a patient according to claim 1. Further aspects of the invention are defined in the dependent claims.

### BRIEF DESCRIPTION OF THE DRAWINGS

These and other embodiments and features will be better understood with reference to the following description and drawings, in which:
FIG. 1 represents a graphical overview of the method according to an embodiment of the present invention;

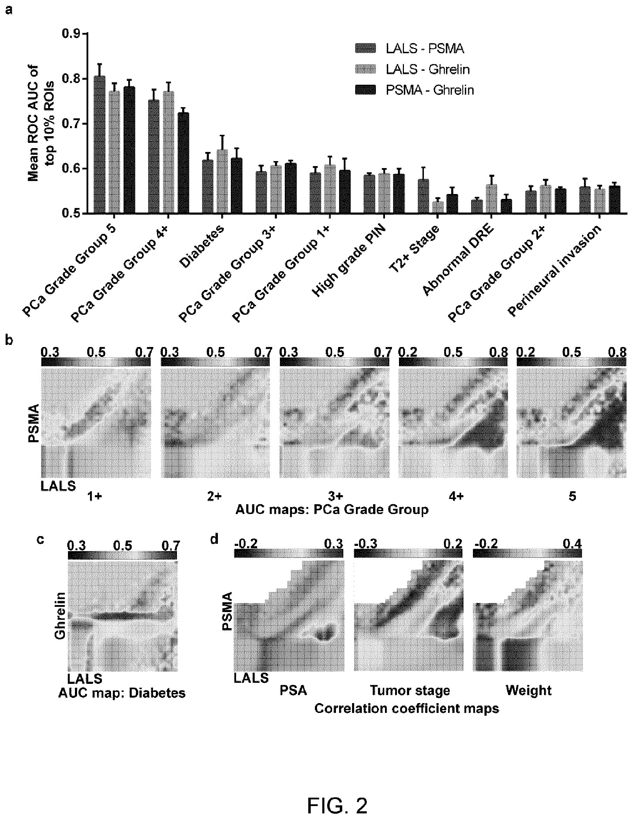

FIG. 2 shows predicting/correlating clinical features using µFCM data. A) receiver operator characteristic area under the curve (ROC AUC) maps for predicting various clinical features using the LALS-PSMA, LALS-ghrelin, and PSMA-ghrelin data sets. The largest 10% AUCs in each map were averaged and compared; B) ROC AUC maps for predicting PCa grade group 1+, 2+, 3+, 4+ and 5 using the LALS-PSMA data set; C) ROC AUC maps for predicting diabetes using the LALS-ghrelin data set; and D) correlation coefficient maps for PSA (right), tumor stage (middle), and weight (right) using the LALS-PSMA data set;
FIG. 3 shows variability of PSMA/ghrelin probe staining on particles from plasma samples complicates conventional manual gating analysis. A), B) and C) are representative scatter plots and ROC AUC maps of large angle light scatter (LALS) and PSMA (a), LALS and ghrelin (b), and PSMA and ghrelin (c) for non-clinically significant and clinically significant PCa patients; D) quantification of PSMA/ghrelin probe positive particles in patient plasma by manual gating; E) ROC curves for predicting clinically significant PCa (grade group 3+) using manual ROI data;
FIG. 4 shows a viSNE analysis of µFCM data. A) Equal number of particles (30,000) from clinically significant and non-clinically significant PCa patients were analyzed with viSNE; B) particles were clustered using the fast search/density peaks algorithm; C) viSNE cluster purity for clinically significant PCa particles. Some clusters show enrichment for particles derived from clinically significant PCa patients (arrow);
FIG. 5 shows optimizing machine learning of µFCM data to predict clinically significant PCa using the PSMA-ghrelin data set; A), B), C) the optimal machine learning algorithm (a), number of bins per optical parameter (b) and number of XG Boost models in an ensemble (c); D) the effect of grid searching XGBoost parameters, feature selection, and ensembling on model performance; E) ROC curves for manual gating, CITRUS, and a custom binning-XGBoost algorithm for predicting clinically significant PCa. Plotted values represent ± SEM with at least 10 repeats of 5-fold cross-validation;

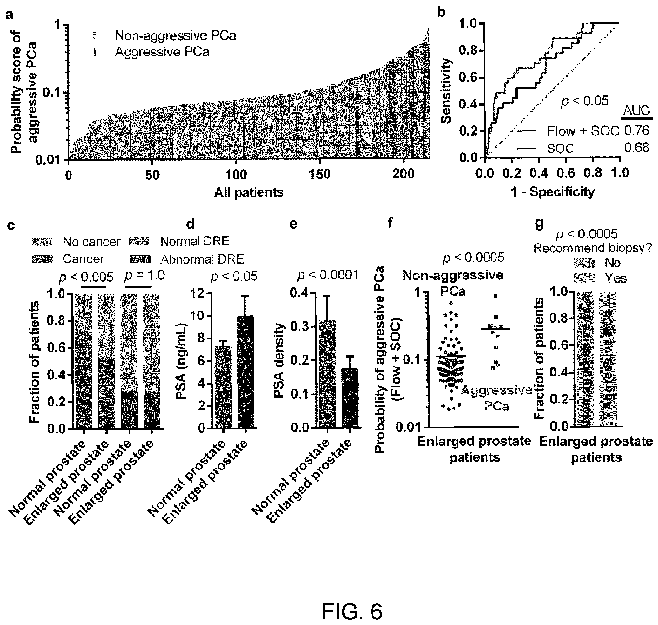

FIG. 6 shows the incorporation of clinical and µFCM data to predict clinically significant PCa. A) Waterfall plot of predictions of clinically significant PCa from a logistic regression model using µFCM-based XGBoost predictions and SOC clinical feature including PSA, age, race, DRE, previous negative biopsy, and family history of PCa; B) Receiver operating characteristic curves of logistic regression models of SOC with or without µFCM data; C) Fraction of patients with or without enlarged prostates (≥40cc) with cancer diagnosis and abnormal DRE; D), E) PSA **(d)** and PSA density (e) in men with and without enlarged prostates. Plotted values are mean ± SEM; F) Predictions of clinically significant PCa in men with enlarged prostates using µFCM + SOC logistic regression model; and G) Recommendation of whether men with enlarged prostates should receive biopsies using µFCM + SOC model; and

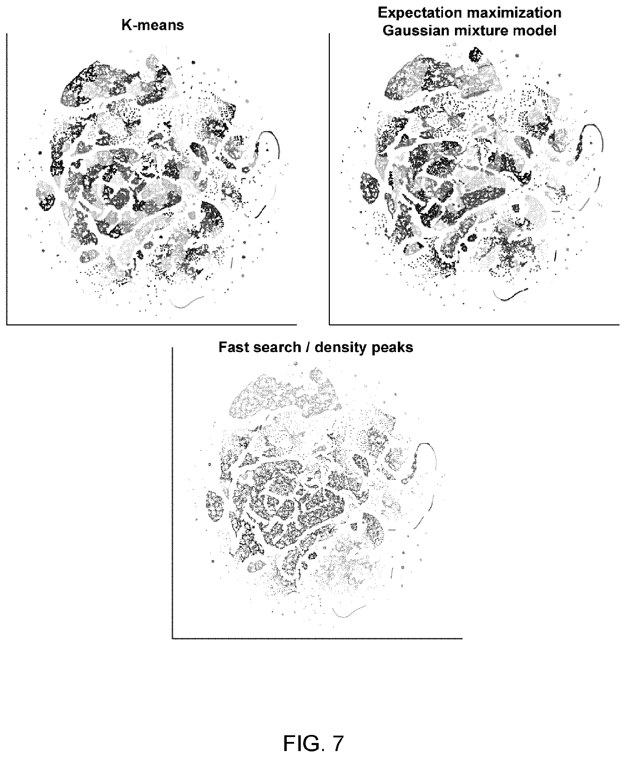

FIG. 7 shows a comparison of clustering algorithms of a viSNE plot of the LALS-PSMA-ghrelin data set. A), B), and C) Clustering algorithms include K-means (a), expectation maximization Gaussian mixture model (b), and fast search/density peaks (c);
FIG. 8 is a graphical representation of XGBoost model performance after PSMA-ghrelin data set transformations;
FIG. 9 shows the variable gain map from XGBoost model using PSMA-ghrelin data set to predict clinically significant PCa (a), and overlay of AUC (color scale) and variable gain (gray scale) maps (b);
FIG. 10 represents a method and results from a highly sensitive detection of single cancer cells using microflow cytometry and ultrasound;
FIG. 11 represents a method and results showing enhanced accuracy of clinical predictions on shifted microflow cytometry data;
FIG. 12 shows biomarker results for Jagged 1;
FIG. 13 shows biomarker results for Cadherin 11, type 2, OB cadherin;
FIG. 14 shows biomarker results for Polysialic acid;
FIG. 15 shows biomarker results for MERTK; and
FIG. 16 shows biomarker results for Prostein.

### DESCRIPTION OF THE INVENTION

Described herein are embodiments illustrative of biomarkers for diagnosing disease, including clinically significant prostate cancer; methods of diagnosing disease, including clinically significant prostate cancer; and methods of developing disease prediction models and diagnostic tests using the same. It will be appreciated that the embodiments and examples described herein are for illustrative purposes intended for those skilled in the art and are not meant to be limiting in any way. All references to embodiments or examples throughout the disclosure should be considered a reference to an illustrative and non-limiting embodiment or an illustrative and non-limiting example.

Unless defined otherwise, all technical and scientific terms used herein have the same meaning as commonly understood by one of ordinary skill in the art to which this invention belongs. It must also be noted that, as used in the specification and the appended claims, the singular forms "a," "an" and "the" include plural referents unless the context clearly dictates otherwise. For example, reference to an "antigen" or "antibody" is intended to include a plurality of antigen molecules or antibodies.

A method of diagnosing disease, such as clinically significant prostate cancer, in a patient is provided. For the purpose of the present discussion, "clinically significant prostate cancer" means a prostate cancer with a Gleason Group 3 or higher. The method involves the steps of: incubating a sample from the patient with one or more probes and/or antibodies which bind one or more biomarkers for the disease of interest; subjecting the sample to microflow cytometry; obtaining signal intensities for the one or more biomarkers and, optionally, obtaining one or more optical properties associated with the sample; processing the signal intensities and, if obtained, the one or more optical properties using custom algorithms to determine the concentration of different particle phenotypes in the patient sample; and diagnosing the patient with the disease based on the output of a machine learning algorithm using particle phenotype concentration data from patient samples as inputs for machine learning. In one embodiment, the disease may be cancer, in particular clinically significant prostate cancer, and the biomarkers correlating to cancer biomarkers, in particular clinically significant prostate cancer biomarkers.

A method of identifying a disease signature is also provided. The method involves the steps of: incubating samples from a healthy subjects and samples from subjects with a known disease with one or more probes which bind to one or more biomarkers for a disease; subjecting the samples to microflow cytometry; obtaining signal intensities for the one or more biomarkers and, optionally, obtaining one or more optical properties associated with each sample; log transforming the signal intensities from the one or more biomarkers and, if present, the one or more optical properties to produce transformed signal intensities; binning particles with similar transformed signal intensities in regions of interest (ROI); determining the concentration of particles in each ROI (which is calculated by dividing the total particle counts in each ROI by the sample volume analyzed during data acquisition), comparing the particle concentration data in each ROI between the samples from healthy subjects and samples from the subjects with a known disease; determining receiver operator characteristic (ROC) area under the curve (AUC) values for each ROI from each combination of markers; and selecting a combination of biomarkers that provides the highest AUC values to obtain the disease signature for the disease.

Samples that are useful in the present invention include, but are not limited to, biological samples, such as blood (or components thereof), semen, milk, etc. In the present invention, extracellular vesicles do not need to be isolated and purified, as is required in other methods. Instead, serum or plasma can be isolated from blood as per standard clinical diagnostic procedures and used, without further purification and processing, in the methods described herein.

The samples are incubated with probes associated with the biomarkers for the disease of interest or the disease being diagnosed, or a particular type of small particle like in this case an EV. Probes can include, but are not limited to, whole antibodies or antibody components such as F(ab), F(ab')₂ or F(ab') fragments, minibodies, etc. against specific antigens, or peptides against specific targets. Probes can also include various dyes that permit identification of particular components in the sample. For example, incubation with lipophilic dyes which stain membrane bound small particles can aid in the segregation of protein aggregates from lipid bound particles in a sample. Typically probes will have a directly conjugated secondary component, such as a fluorescent conjugate, that aids in the detection of the probe bound target.

To detect PSMA positive EVs, the sample can be incubated with the PSMA specific monoclonal antibody J591 (available through BZL Biologics, LLC) which has been directly conjugated with a dye such as DyLight 405. Alternatively a non-conjugated probe (e.g. PSMA specific monoclonal antibody J591), after incubation with sample, may be further incubated with a secondary agent to identify the primary probe used in the assay. For example, incubation with the Qdot565-conjugated donkey anti-mouse IgG antibody, which then permits detection in the µFCM assay. Typically, the biomarker probes will be specific to a biological molecule that is only or primarily expressed in cells or tissues affected by the disease of interest. However, the biomarkers can be specific for a particular cell type. Moreover, more than one biomarker can be used to identify more than one feature of the disease of interest and/or cell type.

Incubation of samples with biomarker probes may be done as a single sample + single probe format, or as a single sample + multiple probe formats. The format of the incubation may provide different answers as each biomarker probe may provide different information on EV populations in a sample. For example, a probe may indicate lipid bound versus non-lipid bound events, or an epithelial versus non-epithelial particle origin. In other cases a probe may indicate disease presence, disease presence and aggressiveness. Multiple probes in an incubation can have similar indications of particle origin, disease presence, and disease aggressiveness. Thus the combination of probes may have significant implications for detection of a disease phenotype.

Particle size and enumeration can be estimated by light scatter. The light scatter characteristics combined with the fluorescence intensity described above can provide a unique phenotype for each particle. These particle phenotypes can be used singular or combined with multiple biomarkers can provided a unique disease signature for the disease of interest.

The samples are then subjected to µFCM, using a commercially available machine, such as, but not limited to, the Apogee A50 microflow cytometer or the CytoFLEX or DxFlex Flow Cytometer. Raw data obtained from the µFCM analysis can be extracted using algorithms, written in MATLAB, R, or Python, and organized as individual particles as rows and light scatter and fluorescence intensities as columns. The time each particle was recorded can be represented in a separate column.

The minimum and maximum cut-offs for light scatter/fluorescence intensity for each particle phenotype can be determined through optimization experiments, which involve using a range of different cut-offs for a range of different light scatter/fluorescence intensities and identifying the cut-offs that provide the highest receiver operator characteristic under the curve from previously acquired patient data.

The number of particles in each particle phenotype can be determined using custom processing scripts which groups particles with similar light scatter and marker intensity. Particle phenotype concentrations are calculated based on particle phenotype counts, the length of time the sample was run, the sample flow rate of the µFCM, and the dilution factor of the sample. If the patient has more than one FCM data file (i.e. multiple replicates), particle phenotype concentrations can be averaged across all replicate µFCM date files.

It is also possible to calculate particle phenotype concentrations in samples using a Dynamic Fluorescence Thresholding algorithm by identifying the biomarker positivity status for each particle in each patient. To determine if particles for each patient sample are positive for a biomarker, a kernel density estimation (KDE) function is applied to the histogram plots of a single probe signal from a single patient sample. The fluorescence value F1 is identified as the region on the X-axis (fluorescence intensity) of the histogram plot that intersects with the highest region on the Y-axis (particle density) on the KDE plot for the biomarker negative particle population, which is the largest peak near the left side of the KDE plot. Next, the slopes of the KDE curve are calculated for many different higher signal intensities from F1. A second fluorescence value, F2, is identified from where the slope is most negative, which is half way down the right side of the negative particle population. The fluorescence intensity value that separates biomarker positive and negative particles (Fs) is equal to F1 + (2 * (F2 - F1)) + F3 where F3 is a small arbitrary fluorescence intensity value that is added to help ensure biomarker negative particles are not classified as biomarker positive particles. Particles with fluorescence intensities above or below Fs are positive or negative for the biomarker, respectively. Once all particles for each patient are classified as positive / negative for each biomarker, the log transformed light scatter signals, used to estimate particle size, are binned into an arbitrary number of groups. Finally, particle phenotypes are created based on all possible combinations of biomarker status (negative / positive) as well as light scatter bin.

From the data collected above, a data set for machine learning is constructed. A table can be created with particle phenotype concentrations for all patients. In one iteration, rows can represent patients and columns represent particle phenotype concentration. However, it will be clear to a person skilled in the art that the data can be represented in an opposite manner or in some other tabular form.

Clinically relevant data can be added as additional columns, or rows depending on how the data set is created, to the table. This data can be used as additional features for machine learning (e.g., does PSA with the µFCM data provide better predictions of who has clinically significant prostate cancer?) or it may be used as labels that the machine learning algorithms need to predict (e.g., identification of which patients have clinically significant prostate cancer).

Once the data set is created, an optimized machine learning model capable of predicting clinical status from µFCM with or without clinical data is generated. Software used for machine learning can include, but is not limited to, R, MATLAB, KNIME, and python. Machine learning models can include single decision tree, support vector machines, k-nearest neighbor, linear regression, logistic regression, discriminant analysis, random forest, neural networks, and XGBoost. The algorithm providing the highest ROC AUC for predicting a clinical condition can be further optimized. All machine learning algorithms are analyzed using 5-fold cross-validation which involves splitting the data into 5 separate groups. A model can be created using 4 of the 5 groups and model accuracy can be determined against the held-out group. The groups are shuffled and the process is repeated 4 more times so that every patient is used once in the held-out group. This ensures model accuracy is determined on data that was not used to create the model.

Machine learning algorithm optimization includes identifying which µFCM / clinical features should be kept / removed before model creation using recursive-feature elimination. This algorithm identifies the most important features from a model using all data (e.g., XGBoost feature importance using the xgb.importance function in R). Multiple data sets are created which include the top 10, 20, 30, 40, 50, 60, 70, 80, 90, or 100% most important features and the data set which provides the highest ROC AUC using 5-fold cross-validation contains the features which will be kept for the final machine learning model. Other feature selection algorithms including genetic algorithms and simulated annealing can also be used at this step.

After feature selection, the tunable parameters of the machine learning algorithm can be optimized through grid searching. This involves providing multiple values for each tunable algorithm parameter (e.g., XGBoost parameters such as "nrounds": 100, 200, and 300 as well as "max_depth": 3, 4, and 5) and testing every combination of possible parameter values. The set of parameter values providing the highest ROC AUC using 5-fold cross-validation is used for the final machine learning model.

The final machine learning model optimization involves ensembling many (typically 2:100) models together by averaging the predictions from all models. All models will use the optimized features and parameters described above, but each model will use a slightly different cohort of patients (e.g., randomly selected 80% of patients) for model creation. This causes each model to be unique and the average of all models' predictions will provide a more accurate and stable prediction of clinical status then using a single model with the full data set. The final optimized ensembled model is saved on a computer for future use.

The final machine learning model can be used to predict clinical status of new patients. New patient data which includes particle phenotype concentrations with or without clinical data can be used as input for the final machine learning model to predict the probability that a patient has a specific clinical condition.

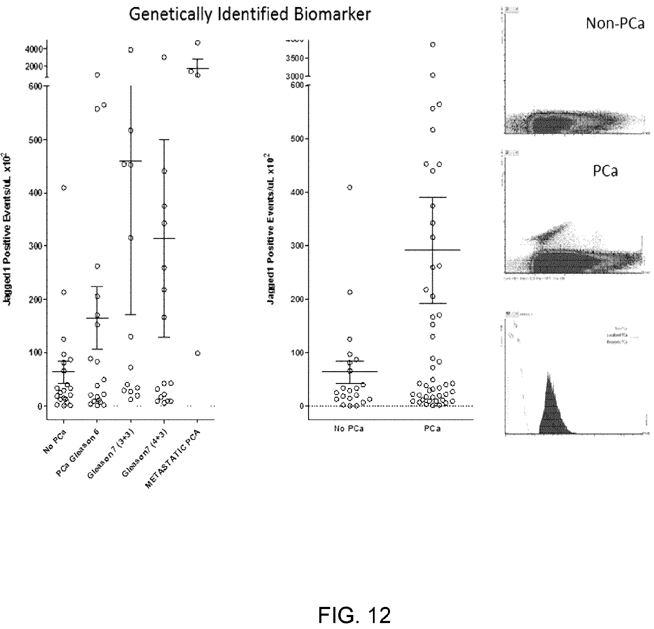

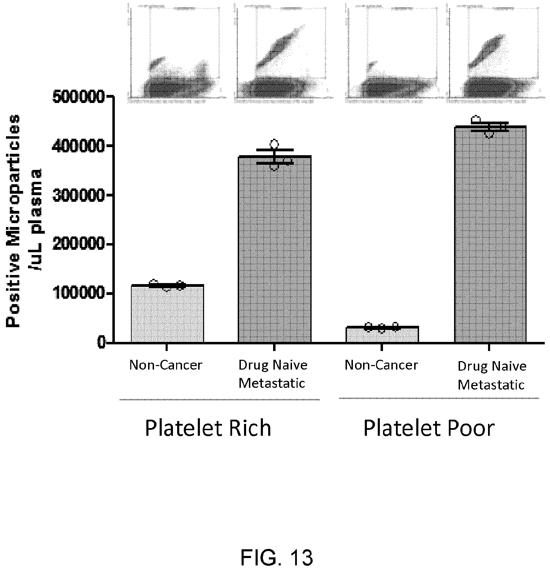

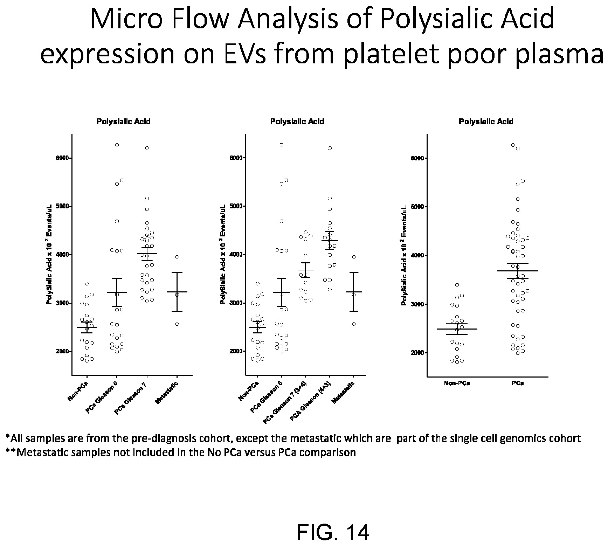

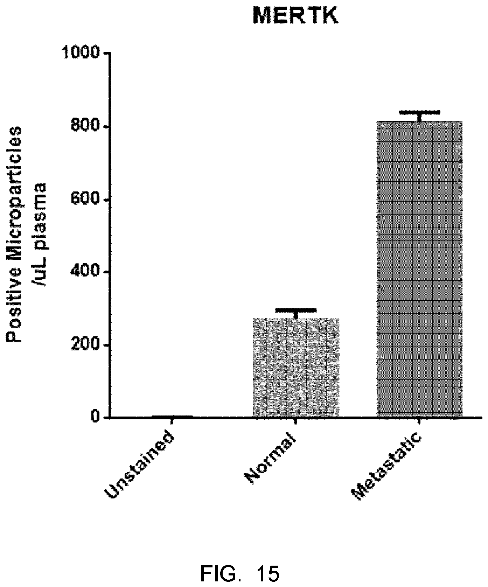

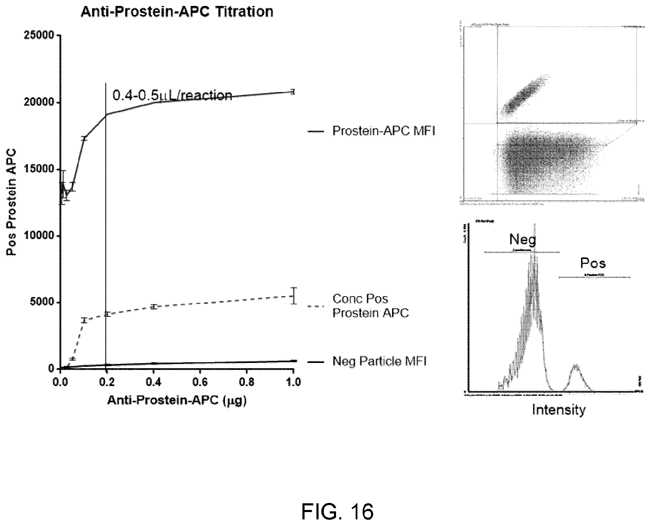

Using the method described above, patients with previously diagnosed clinically significant prostate cancer were studied to determine the particle phenotypes/biomarkers most commonly associated with the disease. These particle phenotypes/biomarkers are shown in Table 1 with additional proof of concept in Figures 12, 13, 14, 15 and 16.

**Table 1: Biomarkers associated with clinically significant prostate cancer**

| GENE | PROTEIN |
|---|---|
| SLC45A3 | Solute carrier 45 member 3 or Prostein |
| FOLH1 | PSMA, prostate specific membrane antigen |
| GHSR | Ghrelin, growth hormone secretagogue receptor (GHSR) |
| JAG1 | jagged 1 |
| CDH11 | cadherin 11, type 2, OB-cadherin (osteoblast) |
| SELE | selectin E |
| MERTK | MER proto-oncogene, tyrosine kinase |
| GABRA2 | gamma-aminobutyric acid (GABA) A receptor, alpha 2 |
| TNFRSF10 B | tumor necrosis factor receptor superfamily, member 10b |
| ABCC5 | ATP-binding cassette, sub-family C (CFTR/MRP), member 5 |
| LETMD1 | LETM1 domain containing 1 |
| CADM1 | cell adhesion molecule 1 |
| EMP2 | epithelial membrane protein 2 |
| ENTPD2 | ectonucleoside triphosphate diphosphohydrolase 2 |
| ABCB11 | ATP-binding cassette, sub-family B (MDR/TAP), member 11 |
| IL17RA | interleukin 17 receptor A |
| RNF122 | ring finger protein 122 |
| ST14 | suppression of tumorigenicity 14 (colon carcinoma) |
| SYPL1 | synaptophysin-like 1 |
| LDLRAD3 | low density lipoprotein receptor class A domain containing 3 |
| HTR1F | 5-hydroxytryptamine (serotonin) receptor 1F, G protein-coupled |
| EMP1 | epithelial membrane protein 1 |
| TRPV6 | transient receptor potential cation channel, subfamily V, member 6 |
| KCNN2 | potassium channel, calcium activated intermediate/small conductance subfamily N alpha, member 2 |
| CLCN6 | chloride channel, voltage-sensitive 6 |
| SLC17A3 | solute carrier family 17 (organic anion transporter), member 3 |
| SLC44A4 | solute carrier family 44, member 4 |
| SLC22A23 | solute carrier family 22, member 23 |
| C9orf91 | chromosome 9 open reading frame 91 |
| RDH10 | retinol dehydrogenase 10 (all-trans) |
| PNKD | paroxysmal nonkinesigenic dyskinesia |
| TMEM229 | transmembrane protein 229B |
| | Sugars |
| | Polysialic Acid (polySA), |
| | neural cell adhesion molecule (N-CAM) |

### EXAMPLES

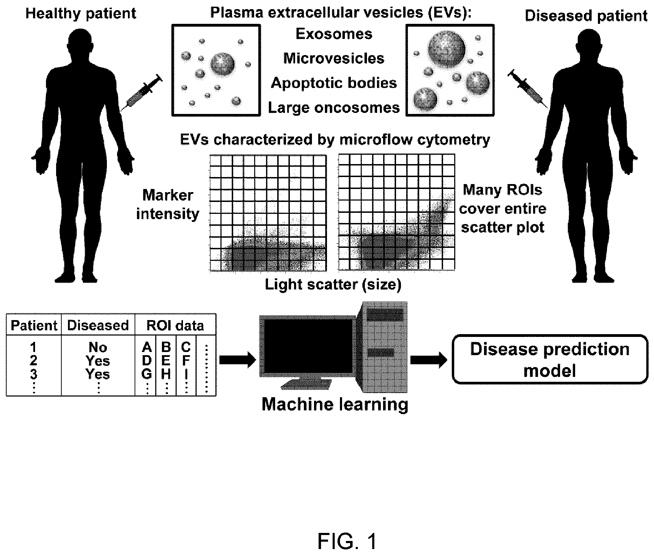

Due to the various sizes of different EVs, the goal was to separate the µFCM data into many different ROIs, where each ROI represents the concentration of different EVs, and use machine learning on the ROI data to predict clinical conditions (Fig. 1). Before creating such models, it was important to first identify which clinical conditions the µFCM data can best predict. Automated analysis scripts were used to create AUC maps of the µFCM data for predicting 10 different clinical conditions which were relevant to the PSMA and ghrelin probes.

When averaging the highest 10% of AUCs within the LALS-PSMA, LALS-ghrelin, and PSMA-ghrelin AUC maps, predicting PCa grade group 5 and 4+ provided the highest averaged AUCs (Fig. 2a). Interestingly, all three bivariate AUCs maps provided top 10% AUCs above 0.7 for predicting these high grade PCa with LALS-PSMA having AUCs above 0.8 for predicting grade group 5 PCa. The LALS-PSMA AUC maps displayed an interesting pattern shift when comparing the different PCa grade groups (Fig. 2b). When estimating particle size using LALS, prediction of grade group 1+ displayed relatively smaller PSMA-positive particles with AUCs above 0.5, meaning particle concentration in these ROIs in general is higher in patients with grade group 1 + PCa, whereas larger PSMA-positive particles mostly displayed AUCs below 0.5, meaning particle concentration in these ROIs in general is lower in patients with grade group 1+ PCa. The AUC maps for higher grade groups demonstrated a progressive inversion of this phenotype with grade group 5 PCa having AUCs >0.8 for larger PSMA-positive particles and AUCs approximately 0.3 for many smaller PSMA-positive particles. This phenotype inversion became quite noticeable with grade group 3+ AUC maps. Previous clinical trials have shown that grade group 3 PCa patients receiving radical prostatectomy had a 10 year recurrence-free progression of under 0.5, which was significantly lower than >0.75 for those patients with grade group 2 PCa (28). This suggests that most men with grade group 3 PCa have metastatic disease at diagnosis since surgical removal of the primary tumor does not cure the patients of PCa. Without being limited by theory, the greater abundance of larger PSMA-positive particles in higher grade PCa patients may be partly due to circulating metastatic cells since larger EVs (>300 nm) from localized tumor cells would have difficulty intravasating into blood vessels.

Due to ghrelin's role in energy and glucose metabolism (Churm R et al., Obes Rev 18(2): 140-148, 2017), AUC maps were created for predicting diabetes. A range of different sized ghrelin-positive particles displayed AUCs near 0.7, suggesting that diabetic men have EVs with elevated levels of ghrelin receptors (Fig. 2c).

Using the LALS-PSMA data, correlation maps were created for PSA, tumor stage, and weight (Fig. 2d). Relatively large particles slightly positive for PSMA demonstrated the highest positive correlation with PSA whereas large particles with strong PSMA positivity correlated best with tumor stage. Such correlations are not surprising since 1) prostate PSMA expression has been shown to correlate with PSA at diagnosis (Kasperzyk JL et al., Cancer Epidemiol Biomarkers Prev 22(12):2354-63, 2013), and 2) higher grade tumors are more likely to spread, explaining the similarity between the higher grade AUC maps and the tumor stage correlation map.

Given the results of the AUC/correlation maps, the µFCM data was used to predict clinically significant PCa which were defined as grade group 3+ since these patients demonstrate significantly worse outcome than grade group 2 and lower PCa patients.

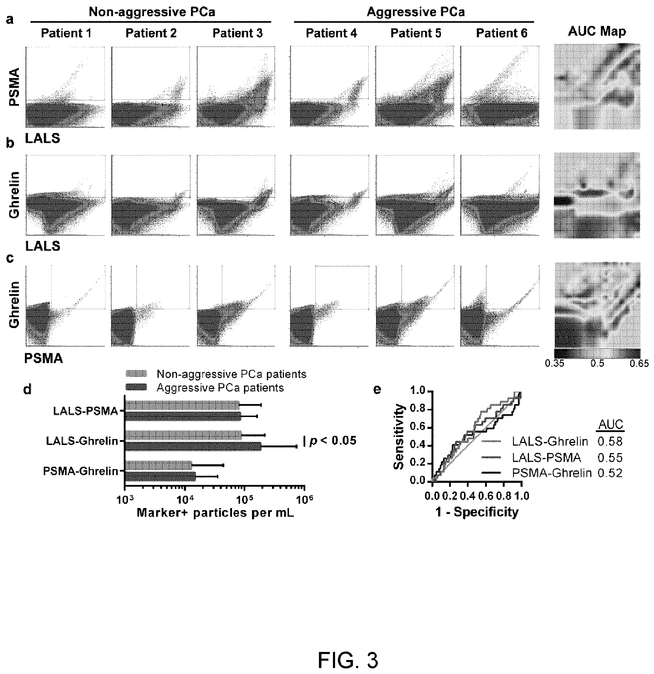

µFCM data was analyzed by manual gating to provide a benchmark of conventional analysis. Creating manual gates around specific particle populations is a non-trivial task since different particle populations exist on different patient scatter plots with some slight shifts in population locations (Fig. 3a, b, c). For simplicity, gates were created that grouped all marker-positive particles. When compared to non-clinically significant PCa, only the concentration of ghrelin-positive particles was significantly higher in clinically significant PCa by 2.1-fold (p *<* 0.05, Fig. 3d). The AUCs of PSMA-, ghrelin-, and PSMA/ghrelin-positive particle concentrations for predicting clinically significant PCa were all below 0.6 (Fig. 3e). These low AUCs may be explained by the AUC maps which show the gates encompassing particles with AUCs above and below 0.5 (Fig. 3a, b, c).

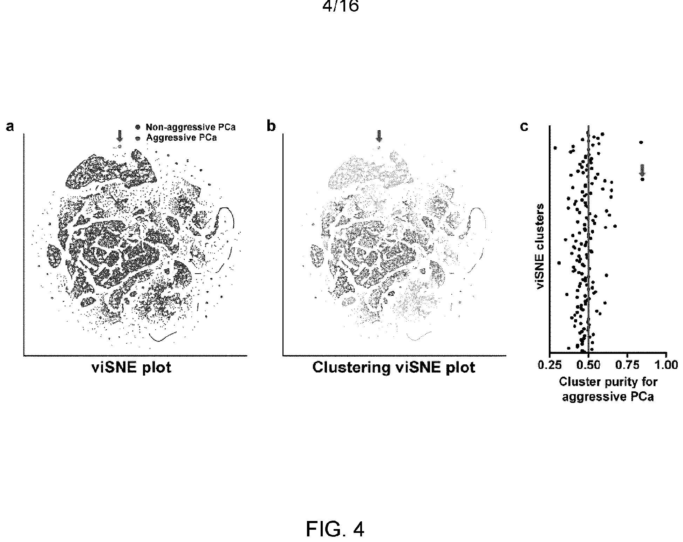

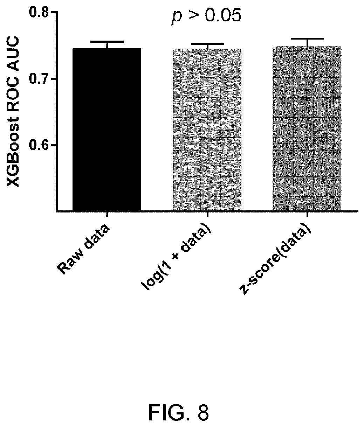

viSNE plots of both clinically significant and non-clinically significant particles together uncovered more particle populations than were visible with conventional scatter plots (Fig. 4a). Particles were clustered using K-means, expectation maximization Gaussian mixture model, and fast search/density peaks algorithms, and the last algorithm was the only one which could maintain large clusters with irregular shapes (Fig. 4b and Fig. 8). Two clusters achieved >0.8 cluster purity for clinically significant PCa, suggesting that these particle populations are in higher levels within clinically significant PCa patients (Fig. 4c). Although these results appear promising to exploit clinically, the non-reproducible nature of viSNE requires all data to be analyzed simultaneously. Since viSNE can only handle up to 100,000 events, >99.99% of particles in the 215 patient cohort would be removed from analysis.

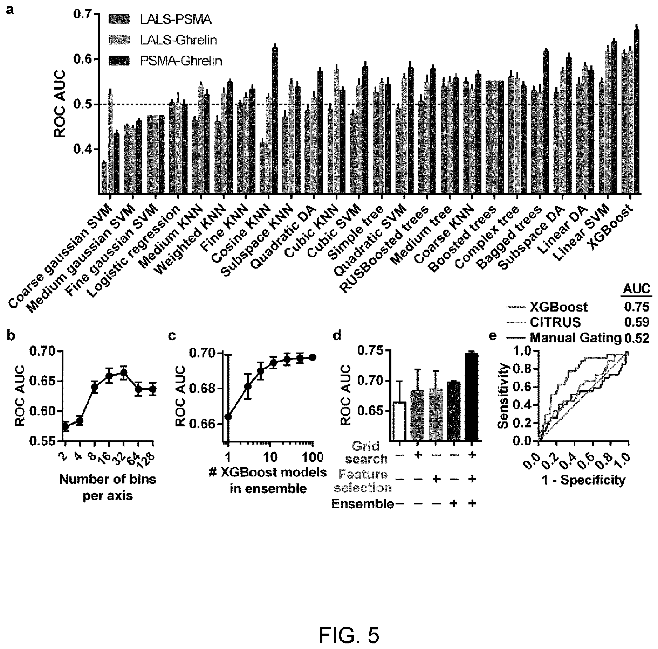

In order to optimize the prediction of clinically significant PCa from µFCM data, particle concentrations from ROIs were used as training data for 24 different machine learning algorithms. For LALS-PSMA, LALS-ghrelin, and PSMA-ghrelin data sets, XGBoost provided the highest AUCs at 0.61, 0.62, and 0.66 (Fig. 5a). All subsequent analysis used the PSMA-ghrelin data set with XGBoost.

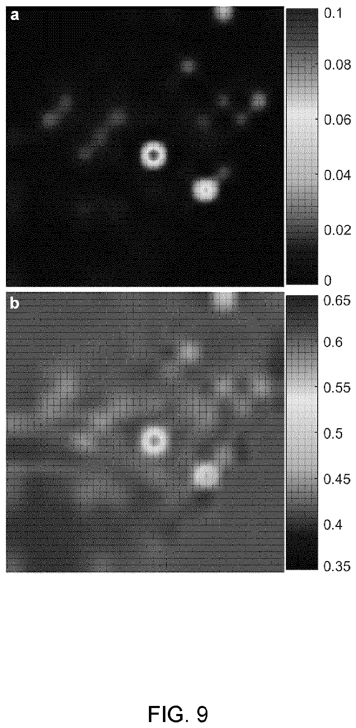

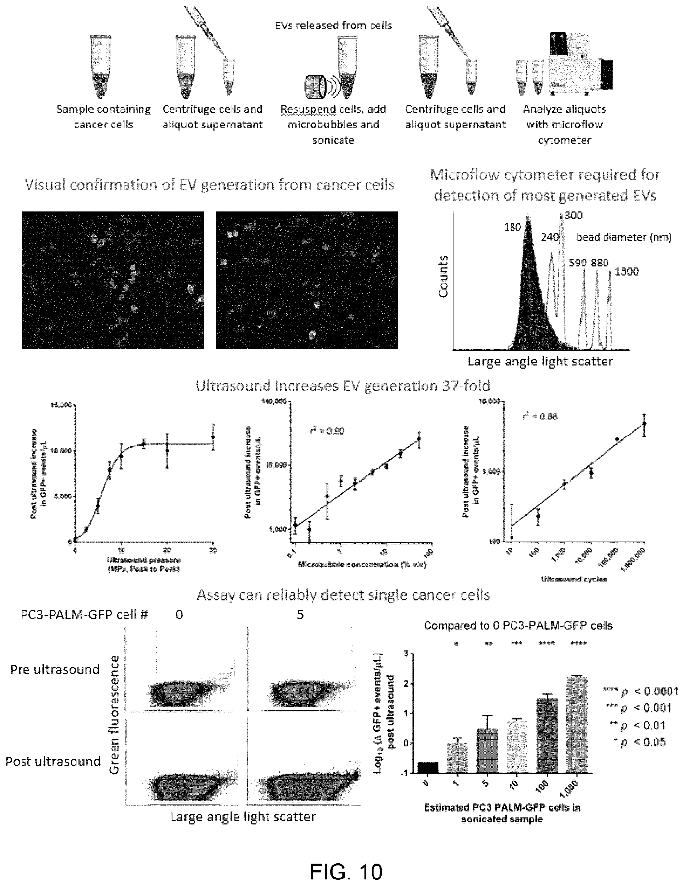

As expected for a decision tree-based model, monotonic transformations of the µFCM data did not improve XGBoost model performance (Fig. 9). The XGBoost variable gain map, which displays the most important ROIs for XGBoost model accuracy, illustrated that many different particle populations are important for the XGBoost model (Fig. 10a). The ROIs with relatively high variable gain mostly overlapped with regions on the AUC map that were well above and below 0.5, suggesting that particle populations which had higher and lower concentrations in clinically significant PCa patients were important for the model (Fig. 10b).

Changing the binning strategy to above or below 32 caused AUCs to decrease, suggesting that this level of resolution is preferred for predicting clinically significant PCa. Creating increasingly larger ensembles of XGBoost models increased model performance (Fig. 5c). Compared to single XGBoost models, an ensemble of 100 models provided a 5% improvement in AUC and reduced model variability by 95%. Larger XGBoost ensembles could be made for greater model performance although such small benefits in accuracy would also have greater processing/memory requirements. Grid searching XGBoost parameters and recursive feature elimination increased XGBoost AUCs by 3% and 5%, respectively (Fig. 5d). Combining grid searching, feature selection, and ensembling significantly increased the XGBoost AUC by 12% (*p* < 0.05), suggesting an additive interaction between model optimization techniques. Citrus and manual gating analysis of the PSMA-ghrelin data set provided significantly lower AUCs, 0.52 and 0.59, respectively, compared to our optimized XGBoost model at 0.75. (*p* < 0.05). The present optimized XGBoost model also outperformed PSA which was the only clinical features which significantly differed between clinically significant and non-clinically significant PCa patients (p = 0.0015, Table 1).

To compare the present optimized model with SOC for predicting clinically significant PCa, logistic regression models were created using SOC with or without our µFCM-based XGBoost model predictions. A waterfall plot of patient predictions from the SOC and µFCM model provided 89% sensitivity and 49% specificity when using a cutoff probability of 0.07332 (Fig. 6a and Table 1). Adding SOC to µFCM predictions slightly increased the AUC to 0.76 which was significantly greater than the 0.68 AUC from SOC alone (p < 0.05), demonstrating the clinical value of the µFCM-based XGBoost model (Fig. 6b).

**Table 2: Patient characteristics by PCa grade group**

| **Patient characteristics by PCa grade group** | **Grade group ≤ 2** | **Grade group ≥3** | ***p*-value** | **ROC AUC mean (CI)** | **Cutoff (>)** | **Sensitivity % (CI)** | **Specificity % (CI)** | **PPV % (CI)** | **NPV % (CI)** |
|---|---|---|---|---|---|---|---|---|---|
| Patients, n | 188 | 27 | | | | | | | |
| Race, n (% black) | 3 (1.6} | 1 (3.7) | 0.42 | 0.51 (0.39-0.63) | - | 3.7 (0.094-19) | 98 (95-100) | 25 (0.63-81) | 88 (82-92) |
| Family history of PCa, n (%) | 53 (29) | 6 (22) | 0.65 | 0.53 (0.42-0.65) | - | 22 (8.6-42} | 71 (64-78) | 10 (3.8-21) | 86 (80-91) |
| Previous negative biopsy, n (%) | 20 (11) | 1 (3.7) | 0.49 | 0.54 (0.42-0.65) | - | 3.7 (0.094-19) | 89 (84-93) | 4.8 (0.12-24) | 86 (81-91) |
| DRE, n (% abnormal) | 48 (26) | 10 (37) | 0.25 | 0.56 (0.44-0.68) | - | 37 (19-58) | 74 (67-80) | 17 (8.6-29) | 89 (83-93) |
| Age, γr, mean (CI) | 62 (60-63) | 65 (61-68) | 0.2 | 0.58 (0.45-0.70) | 53.95 | 89 (71-98) | 14 (9.7-20) | 13 (8.5-19) | 90 (73-98) |
| PSA, ng/ml, mean (CI) | 7.4 (6.2-8.7} | 16 (3.8-29) | 0.0015 | 0.69 (0.59-0.79) | 5.25 | 89 (71-98) | 42 (35-49) | 18 (12-26) | 96 (90-99) |
| SOC score | 12 (11-13) | 17 (11-23) | 0.0023 | 0.68 (0.57-0.79) | 9.472 | 89 (71-98) | 30 (24-37) | 15 (10-22) | 95 (86-99) |
| Flow assay score, mean (CI) | 35 (34-36) | 40 (37-42) | <0.0001 | 0.75 (0.66-0.84) | 32.59 | 89 (71-98) | 48 (41-56) | 20 (13-28) | 97 (91-99) |
| Flow assay + SOC score, mean (CI) | 11(9-12) | 24 (16-32) | <0.0001 | 0.76 (0.67-0.86) | 7.332 | 89 (71-98) | 49 (42-56) | 20 (13-28) | 97 (91-99) |

DRE, digital rectal exam; SOC, standard of care; CI, 95% confidence interval; ROC AUC, receiver operator characteristic area under the curve; PPV, positive predictive value; NPV, negative predictive value;

Upon further analysis of the 215 patient cohort, it was observed that men with enlarged prostates (>40 cc) were significantly less likely to have PCa, meaning that compared to men with normal sized prostates, a greater percentage of men with enlarged prostates underwent unnecessary biopsies. Based on current clinical practice, men primarily receive prostate biopsies due to high PSA levels and/or abnormal DRE. The fraction of patients with abnormal DRE was similar between men with normal and enlarged prostates (Fig. 6c) while PSA levels were significantly higher in men with enlarged prostates (p < 0.05, Fig. 6d), suggesting that elevated PSA was responsible for the increased number of unnecessary biopsies. Normalizing PSA levels using PSA density (PSA divided by prostate volume) may not be ideal since PSA density was significantly lower in men with enlarged prostate (Fig. 6e). For men with enlarged prostates, the SOC + µFCM probability scores for clinically significant PCa were significantly different between non-clinically significant and clinically significant PCa patients (p < 0.0005, Fig. 6f), and using the previously define probability cutoff threshold in Table 2, 100% and 49% of patients with clinically significant and non-clinically significant PCa would be recommended for biopsy, respectively, eliminating approximately half of unnecessary biopsies while still maintain 100% sensitivity for detecting clinically significant PCa (Fig. 6g).

### A. Patient characteristics and sample acquisition

Pre-biopsy plasma samples were acquired from the Alberta Prostate Cancer Research Initiative (APCaRI) biorepository. The inclusion criteria were adult men without prior prostate cancer diagnosis who were: (1) referred to urology clinics in Alberta for prostate concerns and were being scheduled for a prostate biopsy; and (2) undergoing transurethral prostate surgery for diagnosis or treatment of prostate abnormalities. All patients provided written informed consent, and the study was approved by the scientific ethics committees at the Prostate Cancer Centre (Calgary, Alberta, Canada) and the Northern Alberta Urology Centre (Edmonton, Alberta, Canada). Patients were enrolled between June 2014 and September 2015. Transrectal ultrasound guided prostate biopsies were performed with a median of 12 cores per patient and evaluated according to each hospital's SOPs. Test results were not provided to the clinical sites for patient care. Laboratory personnel who acquired patient samples and ran tests with them were blinded for patient characteristics. Blood was collected and processed to collect plasma as per institutional SOPs and time from arm to -80°C freezer was 2 hours or less. In particular, blood samples were collected in clinical grade vacutainers. For plasma preparation, samples underwent a 2 step centrifugation process. First a standard 1300 xg for 10 minutes to provide separation of plasma from other blood components followed by a second 1300 xg x 10 minutes centrifugation to pellet platelets. Blood collected in serum tubes is first allowed to clot for 15-30 minutes and then a single 1300 xg x 10 minutes centrifugation step is performed.

### B. µFCM assay

Frozen plasma samples were thawed, centrifuged at 16,000 x g for 30 minutes to remove large debris and platelet particles, and incubated with 400 µg/mL J591 antibody and 1/50 final dilution of secondary Qdot565-conjugated donkey anti-mouse IgG antibody. Samples were also incubated with 0.025 mM Ghrelin Cy5 probe containing the first 18 amino acids of ghrelin. Thirty minutes after probe incubation, samples were diluted 100-fold in double filtered (0.22 µm) phosphate buffered saline and analyzed with the Apogee A50 microflow cytometer using a flow rate of 3.01 µL/minute. Samples were run for up to 2 minutes or until 5,000,000 events were recorded, whichever came first. Plasma from each patient was run in triplicate. Conventional manual gating analysis of µFCM data was performed using Histogram version 255.0.0.80 software (Apogee Flow Systems).

### C. Processing µFCM data

Patient µFCM fcs files were analyzed using a custom MATLAB (version R2017a) script. Within each fcs file, signal intensities for all channels were log transformed and particles with similar optical properties were binned using 32-bins per optical property unless stated otherwise. Three different bivariate histograms of particle concentration were created: 1) large angle light scatter (LALS) and PSMA stain intensity, 2) LALS and ghrelin probe stain intensity, and 3) PSMA and ghrelin probe stain intensity. Each bivariate histogram contained 1024 ROls (32x32 bins). Particle concentration in each ROI was averaged over the three replicates per patient.

### D. Predicting and correlating clinical features with µFCM data

The µFCM data was used to predict binary clinical features (e.g., patients with or without diabetes, normal or abnormal digital rectal exam) and correlate with ordinal or interval clinical features (e.g., tumor stage or PSA, respectively) using a custom MATLAB script. To minimize the code needed for automated analysis, an excel instruction file was created which described how the µFCM data should be analyzed for each clinical feature. Within the instruction file, each clinical feature was a separate column and each row contained specific information or instructions. Specific information included the location of the clinical feature within the database, the type of data for each clinical feature (binary or ordinal/interval), and the value which represents missing data for that clinical feature. Instructions primarily involved how the clinical feature should be transformed which included thresholding values when binarizing features, deriving the PCa grade groups from Gleason scores, and determining age from dates of birth. Patients missing data for the clinical feature were removed from analysis for that clinical feature.

Once clinical feature data was retrieved from the database for all patients and transformed, µFCM particle concentration data for each ROI was used to predict or correlate with clinical features. For binary clinical features, receiver operator characteristic (ROC) area under the curve (AUC) values were determined for each ROI and AUC maps were generated for each bivariate data set including LALS-PSMA, LALS-ghrelin, and PSMA-ghrelin. For ordinal/interval clinical features, Pearson correlation coefficients were determined for each ROI and correlation maps were generated for each bivariate data set. The highest 10% of AUC values in each AUC map were averaged and these values were compared across clinical features.

### E. viSNE analysis of µFCM data

viSNE plots were created using Cyt version 2.0 software run on MATLAB (25). Each patient's triplicate fcs files were concatenated into one fcs file. Two new fcs files were created: one using events from patients with grade group 2 and lower PCa (non-clinically significant PCa), and the other using events from patients with grade group 3 and higher PCa (clinically significant PCa). These two fcs files had a total of approximately 100,000 events with an equal number of events from each patient within their group. With Cyt software, 30,000 events from both of these two fcs files were randomly subsampled and merged to create 60,000 events which were visualized with viSNE using the bh-SNE transformation using LALS, PSMA, and ghrelin channels and clustered with the k-means and expectation maximization Gaussian mixture model algorithms. The viSNE results were exported from Cyt and also clustered using the fast search / density peaks algorithm using the DensityClust function for Matlab (Rodriguez A and Laio A, Science 344(6191):1492-6, 2014). Event pair Euclidean distances were determined using the pdist2 function. For setting delta and rho parameters using the paraSet function, the percent neighbor variable was set to 2% and a Gaussian kernel was used. Cluster centers were selected using delta values between 1.5 and 5 as well as rho values between 200 and 1900. For all clustering algorithms, 248 clusters were created over the 60,000 events. Cluster purity for clinically significant PCa was defined as the number of clinically significant PCa events divided by the total number events within each cluster. Only clusters with at least 60 particles (0.1% of total particles) were analyzed.

### F. Optimizing machine learning models for predicting clinically significant PCa

MATLAB's classification learner app was used to test 23 different machine learning algorithms to predict clinically significant PCa using particle concentration µFCM data. These algorithms included individual/bagged/boosted decision trees, linear/quadratic/cubic/Gaussian support vector machines, logistic regression, linear/quadratic/subspace discriminant analysis, and k-nearest neighbors. XGBoost was also tested using the 'xgboost' package in R (version 3.3.3). All machine learning algorithms used default settings and 5-fold cross-validation repeated at least 10 times with patient randomization between repeats.

The machine learning algorithm with the highest AUC was then optimized by 1) comparing 2, 4, 8, 16, 32, 64, and 128 bins when processing the µFCM data, 2) creating ensembles of 3, 6, 12, 25, 50, and 100 models using the same machine learning algorithm but randomly selecting different subsets of patients as training data and averaging model predictions, 3) selecting the best subset of µFCM ROIs using recursive feature elimination with the R 'caret' package, and 4) grid searching algorithm parameters (XGBoost: nrounds = 50, 100, 150, 200, 250, 300, 400; max_depth = 3, 4, 5, 6; eta = 0.01, 0.1; gamma = 0; colsample_bytree = 1; min_child_weight = 1; subsample = 1). The binning/ensembling/features/parameters that provided the highest AUCs were used together to create a final model for predicting clinically significant PCa. This model was compared to manual gating analysis using Histogram software and Citrus with default settings using R. Citrus predicts clinical conditions from flow cytometry data by using hierarchical clustering and lasso-regularized logistic regression and nearest shrunken centroid methods (Bruggner RV et al., Proc Natl Acad Sci USA 111(26):E2770-7, 2014).

To incorporating standard of care (SOC) clinical features, including PSA, age, DRE, family history of PCa, previous negative biopsy, and race (black = 1, other races = 0), with the final µFCM model probability predictions, a logistic regression model was created using all of these features. This model was compared to a similar logistic regression model without using µFCM data.

### G. Statistical analysis

Unless stated otherwise, bar/dot plots with error bars represent mean ± standard error of the mean. When comparing 2 groups, unpaired two-tailed t-tests were used for interval data and Fisher's exact tests were used for contingency tables. One-way ANOVA was used for comparing 3 or more groups using Tukey's multiple comparison test. ROC curves were compared by DeLong's method using the 'pROC' package in R. When possible, ROC cut-off values were determined using ~90% sensitivity and the resulting specificity and positive/negative predictive values were determined using GraphPad Prism version 6.01 software.

### Development of an assay for circulating tumor cells detection by analysing EVs from sonicated samples using microflow cytometry

As shown in FIG. 10, for assay optimization, 100,000 HeLa-GFP cells in medium were incubated and exposed to various amounts of microbubbles and ultrasound. Medium was analyzed for fluorescent Evs before and after sonication. For testing assay sensitivity, 0, 1, 5, 10, 100, and 1,000 PC3 prostate cancer cells expressing palmitoylated GFP were mixed with 1,000,000 HT1080 cells (background) and were sonicated with microbubbles. Medium was analyzed by microflow cytometry pre/post sonication.

The assay has single cancer cell detection with higher theoretical SNR than conventional flow cytometry. Ultrasound ≥15 Mpa pressure generates maximal EVs. Ultrasoundmediated EV release linearly increases with ultrasound cycles and microbubble concentration.

### Development of algorithms to standardize light scatter signals and fluorescence signals

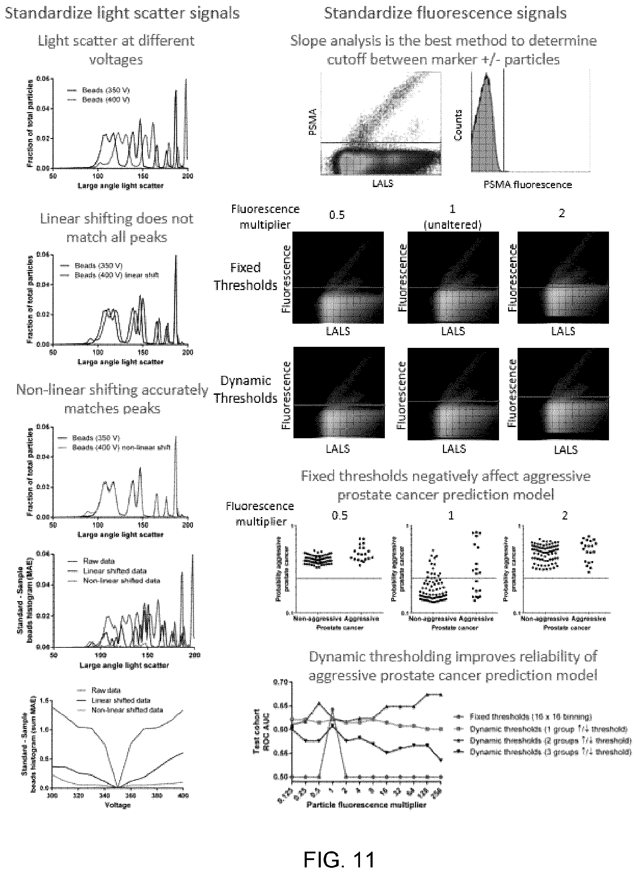

As shown in FIG. 11, light scatter signals were recorded from calibration beads under different voltages (300 - 400 V in +10 V). Light scatter histograms were created and linearly shifted to match beads run at 350 V. Each bead histogram peak was shifted to match the same peak of beads run at 350 V (non-linear shifting).

Plasma samples from 281 patients were stained with prostate-specific membrane antigen. Data was processed with fixed 16 x 16 binning (LALS vs PSMA) or using an algorithm that identified particles positive or negative for PSMA using dynamic fluorescence thresholding and separated groups further based on degree of PSMA positivity. XGBoost models were created to predict patients with aggressive prostate cancer (Gleason 4+3 and higher). Models were used on the same data with modified fluorescence (x 0.125 - 256) and AUCs were calculated.

The non-linear light scattering calibration algorithm can help correct light scatter variability in samples. Processing data with dynamic fluorescence thresholds helps ensure model reliability on shifted data. These standardization algorithms will improve clinical assay predictions when used in many clinics over time.

## Claims

1. A method of diagnosing disease signature for a disease in a patient, the method comprising the steps of:
incubating a sample comprising extracellular vesicles (EVs) from the patient with one or more probes that bind biomarkers for the disease of interest bound to the EVs;
subjecting the sample to microflow cytometry to identify the EVs;
obtaining signal intensities for the one or more biomarkers and, obtaining one or more optical properties associated with the sample;
processing the signal intensities and, if obtained, the one or more optical properties to calculate concentrations of different particle phenotypes in the sample; wherein the processing comprises log transforming the signal intensities to produce transformed signal intensities; and binning particles with similar transformed signal intensities into regions of interest (ROI) for each optical property where each ROI is considered a different particle phenotype; and
calculating particle phenotype concentrations in samples using a Dynamic Fluorescence Thresholding algorithm by identifying the biomarker positivity status for each particle in each patient;
using these concentrations of particle phenotypes as inputs for machine learning algorithms to determine the probability of patients having clinically significant prostate cancer.

2. The method of claim 1, wherein the log transforming and binning steps occur simultaneously or separately.

3. The method of claim 2, wherein binning the particles comprises binning using a set number of bins per optical property.

4. The method of any one of claims 1-3, wherein the method comprises a plurality of ROIs.

5. The method of claim 1, wherein the Dynamic Fluorescence Thresholding algorithm identifies the biomarker positivity status for each particle in each patient by:
fitting a kernel density estimation (KDE) function is to the particle signal data for all particles each biomarker;
identifying the fluorescence value F1 which intersects the highest region on the Y-axis (particle density) on the KDE plot for the biomarker negative particle population;
calculating slopes on the KDE curve for many different higher fluorescent signal intensities from F1 to identify a second fluorescence value, F2, which is where the slope is mostly negative;
calculating the fluorescence intensity value that separates biomarker positive and negative particles (Fs) which is equal to F1 + (2 * (F2 - F1)) + F3 where F3 is a small arbitrary fluorescence intensity value that is added to ensure biomarker negative particles are not classified as biomarker positive particles;
determining the biomarker positivity status of all particles based on if the particles have biomarker signal above (biomarker positive) or below (biomarker negative) Fs;
binning particles into different estimated size groups based on their light scatter intensities; and
determining particle phenotypes by all possible combinations of biomarker positivity and light scatter groups.

6. The method of any one of claims 1 to 5, wherein the machine learning algorithm is an individual/bagged/boosted decision tree algorithm, linear/quadratic/cubic/Gaussian support vector machine algorithm, logistic regression, linear/quadratic/subspace discriminant analysis, or k-nearest neighbors algorithm, preferably the machine learning algorithm is a boosted decision tree algorithm, more preferably the machine learning algorithm is XGBoost algorithm.

7. The method of claim 6, wherein the extreme gradient boosted decision tree algorithm comprises an ensemble of at least 100 models with output probabilities averaged.

8. The method of claim 1, wherein the one or more biomarkers are selected from the group consisting of SLC45A3, FOLH1, GHSR, JAG1, CDH11, SELE, MERTK, GABRA2, TNFRSF10B, ABCC5, LETMD1, CADM1, EMP2, ENTPD2, ABCB11, IL17RA, RNF122, ST14, SYPL1, LDLRAD3, HTR1F, EMP1, TRPV6, KCNN2, CLCN6, SLC17A3, SLC44A4, SLC22A23, C9orf91, RDH10, PNKD, and TMEM229.

9. The method of any one of claims 1 to 8, wherein the sample is a serum, plasma, urine or semen sample.

10. The method of claim 1, wherein a mixture of probes is used that bind tissue specific biomarkers, cancer specific biomarkers, outcome specific biomarkers, or a combination thereof, preferably
wherein the tissue specific biomarker is a prostate specific biomarker, or
wherein the cancer specific biomarker is ghrelin, or
wherein the outcome specific biomarker is polysialic acid.

## Patentansprüche

1. Verfahren zur Diagnose einer Krankheitssignatur für eine Krankheit bei einem Patienten, wobei das Verfahren die folgenden Schritte umfasst:
Inkubieren einer Probe, die extrazelluläre Vesikel (EVs) des Patienten umfasst, mit einer oder mehreren Sonden, die Biomarker für die Krankheit von Interesse binden, die an die EVs gebunden sind;
Aussetzen der Probe einer Mikrodurchflusszytometrie, um die EVs zu identifizieren;
Erhalten von Signalintensitäten für den einen oder die mehreren Biomarker und Erhalten von einer oder mehreren optischen Eigenschaften, die mit der Probe assoziiert sind;
Verarbeiten der Signalintensitäten und, falls erhalten, der einen oder mehreren optischen Eigenschaften, um Konzentrationen von verschiedenen Partikelphänotypen in der Probe zu berechnen; wobei das Verarbeiten ein logarithmisches Transformieren der Signalintensitäten umfasst, um transformierte Signalintensitäten zu produzieren; sowie Binning von Partikeln mit ähnlichen transformierten Signalintensitäten in Regionen von Interesse (ROI) für jede optische Eigenschaft, wobei jede ROI als ein anderer Partikelphänotyp angesehen wird; und
Berechnen von Partikelphänotypkonzentrationen in Proben mittels eines dynamischen Fluoreszenzschwellenwertalgorithmus durch Identifizieren des Biomarkerpositivitätsstatus für jedes Partikel in jedem Patienten;
Verwenden dieser Konzentrationen der Partikelphänotypen als Eingaben für Maschinenlernalgorithmen, um die Wahrscheinlichkeit zu bestimmen, dass Patienten klinisch signifikanten Prostatakrebs aufweisen.

2. Verfahren nach Anspruch 1, wobei die Schritte des logarithmischen Transformierens und des Binnings gleichzeitig oder getrennt erfolgen.

3. Verfahren nach Anspruch 2, wobei das Binning der Partikel ein Binning mittels einer festgelegten Anzahl an Bins pro optischer Eigenschaft umfasst.

4. Verfahren nach einem der Ansprüche 1-3, wobei das Verfahren eine Vielzahl von ROIs umfasst.

5. Verfahren nach Anspruch 1, wobei der dynamische Fluoreszenzschwellenwertalgorithmus den Biomarkerpositivitätsstatus für jedes Partikel in jedem Patienten durch Folgendes identifiziert:
Anpassen einer Kerndichteschätzungs(KDE-)Funktion an die Partikelsignaldaten für alle Partikel jedes Biomarkers;
Identifizieren des Fluoreszenzwertes F1, der die höchste Region auf der Y-Achse (Partikeldichte) auf dem KDE-Diagramm für die Biomarkernegativpartikelpopulation schneidet;
Berechnen von Steigungen auf der KDE-Kurve für viele verschiedene höhere Fluoreszenzsignalintensitäten von F1, um einen zweiten Fluoreszenzwert F2 zu identifizieren, der dort liegt, wo die Steigung meist negativ ist,
Berechnen des Fluoreszenzintensitätswertes, der Biomarkerpositivpartikel und Biomarkernegativpartikel (Fs) trennt, der gleich F1 + (2 * (F2 - F1)) + F3 ist, wobei F3 ein kleiner willkürlicher Fluoreszenzintensitätswert ist, der hinzugefügt wird, um sicherzustellen, dass Biomarkernegativpartikel nicht als Biomarkerpositivpartikel klassifiziert werden;
Bestimmen des Biomarkerpositivitätsstatus von allen Partikeln basierend darauf, ob die Partikel ein Biomarkersignal über (Biomarkerpositiv) oder unter (Biomarkernegativ) Fs aufweisen;
Binning von Partikeln in verschiedene geschätzte Größengruppen basierend auf ihren Lichtstreuungsintensitäten; und
Bestimmen von Partikelphänotypen durch alle möglichen Kombinationen von Biomarkerpositivitäts- und Lichtstreuungsgruppen.

6. Verfahren nach einem der Ansprüche 1 bis 5, wobei der Maschinenlernalgorithmus ein individueller/verpackter/geboosterter Entscheidungsbaumalgorithmus, linearer/quadratischer/kubischer/Gaußscher Supportvektor-Maschinenalgorithmus, logistische Regression, lineare/quadratische/Subraum-Diskriminanzanalyse oder k-nächster-Nachbar-Algorithmus ist, bevorzugt wobei der Maschinenlernalgorithmus ein geboosterter Entscheidungsbaumalgorithmus ist, mehr bevorzugt wobei der Maschinenlernalgorithmus ein XGBoost-Algorithmus ist.

7. Verfahren nach Anspruch 6, wobei der extreme gradientengeboosterte Entscheidungsbaumalgorithmus ein Ensemble von zumindest 100 Modellen mit gemittelten Ausgangswahrscheinlichkeiten umfasst.

8. Verfahren nach Anspruch 1, wobei der eine oder die mehreren Biomarker ausgewählt sind aus der Gruppe bestehend aus SLC45A3, FOLH1, GHSR, JAG1, CDH11, SELE, MERTK, GABRA2, TNFRSF10B, ABCC5, LETMD1, CADM1, EMP2, ENTPD2, ABCB11, IL17RA, RNF122, ST14, SYPL1, LDLRAD3, HTR1F, EMP1, TRPV6, KCNN2, CLCN6, SLC17A3, SLC44A4, SLC22A23, C9orf91, RDH10, PNKD und TMEM229.

9. Verfahren nach einem der Ansprüche 1 bis 8, wobei die Probe eine Serum-, Plasma-, Urinoder Samenprobe ist.

10. Verfahren nach Anspruch 1, wobei eine Mischung von Sonden verwendet wird, die gewebespezifische Biomarker, krebsspezifische Biomarker, ergebnisspezifische Biomarker oder eine Kombination davon binden, bevorzugt
wobei der gewebespezifische Biomarker ein prostataspezifischer Biomarker ist, oder
wobei der krebsspezifische Biomarker Ghrelin ist, oder
wobei der ergebnisspezifische Biomarker Polysialinsäure ist.

## Revendications

1. Procédé de diagnostic de signature de maladie d'une maladie chez un patient, le procédé comprenant les étapes de :
incubation d'un échantillon comprenant des vésicules extracellulaires (VE) du patient avec une ou plusieurs sondes qui lient des biomarqueurs de la maladie en question liées aux VE ;
soumission de l'échantillon à une cytométrie en microflux pour identifier les VE ;
obtention d'intensités de signal pour le ou les biomarqueurs et obtention d'une ou plusieurs propriétés optiques associées à l'échantillon ;
traitement des intensités de signal et, si elles sont obtenues, de la ou des propriétés optiques pour calculer des concentrations de différents phénotypes de particules dans l'échantillon ; ledit traitement comprenant la transformation logarithmique des intensités de signal pour produire des intensités de signal transformées ; et regroupement de particules possédant des intensités de signal transformées similaires en régions d'intérêt (ROI) pour chaque propriété optique, où chaque ROI est considérée comme un phénotype de particule différent ; et
calcul de concentrations de phénotypes de particules dans des échantillons à l'aide d'un algorithme de seuillage de fluorescence dynamique en identifiant l'état de positivité de biomarqueurs pour chaque particule chez chaque patient ;
utilisation de ces concentrations de phénotypes de particules comme intrants pour les algorithmes d'apprentissage automatique afin de déterminer la probabilité que des patients aient un cancer de la prostate cliniquement significatif.

2. Procédé de la revendication 1, dans lequel les étapes de transformation logarithmique et de regroupement se produisent simultanément ou séparément.

3. Procédé de la revendication 2, dans lequel le regroupement des particules comprend le regroupement en utilisant un nombre défini de fichiers par propriété optique.

4. Procédé de l'une quelconque des revendications 1 à 3, ledit procédé comprenant une pluralité de ROI.

5. Procédé de la revendication 1, dans lequel l'algorithme de seuillage de fluorescence dynamique identifie l'état de positivité de biomarqueurs pour chaque particule chez chaque patient par :
l'ajustement d'une fonction d'estimation de densité de noyau (KDE) aux données de signal de particules pour toutes les particules de chaque biomarqueur ;
l'identification de la valeur de fluorescence F1 qui coupe la région la plus élevée sur l'axe Y (densité de particules) sur le graphique KDE pour la population de particules négatives de biomarqueurs ;
le calcul des pentes sur la courbe KDE pour de nombreuses intensités de signal fluorescent différentes plus élevées à partir de F1 afin d'identifier une seconde valeur de fluorescence, F2, où la pente est principalement négative ;
le calcul de la valeur d'intensité de fluorescence qui sépare des particules positives et négatives de biomarqueurs (Fs) qui est égale à F1 + (2 * (F2 - F1)) + F3 où F3 est une petite valeur d'intensité de fluorescence arbitraire qui est ajoutée pour s'assurer que des particules négatives de biomarqueurs ne sont pas classées comme des particules positives de biomarqueurs ;
la détermination de l'état de positivité de biomarqueurs de toutes les particules sur la base du fait qu'un signal de biomarqueur des particules est supérieur (biomarqueur positif) ou inférieur (biomarqueur négatif) à Fs ;
le regroupement de particules en différents groupes de taille estimée sur la base de leurs intensités de diffusion de la lumière ; et
la détermination de phénotypes de particules par toutes les combinaisons possibles de la positivité de biomarqueurs et de groupes de diffusion de la lumière.

6. Procédé de l'une quelconque des revendications 1 à 5, dans lequel l'algorithme d'apprentissage automatique est un algorithme d'arbre de décision individuel/en sac/amplifié, un algorithme de machine à vecteurs de support linéaire/quadratique/cubique/gaussienne, une régression logistique, une analyse discriminante linéaire/quadratique/de sous-espace, ou un algorithme des k plus proches voisins, de préférence l'algorithme d'apprentissage automatique est un algorithme d'arbre de décision amplifié, idéalement l'algorithme d'apprentissage automatique est un algorithme XGBoost.

7. Procédé de la revendication 6, dans lequel l'algorithme d'arbre de décision à gradient amplifié extrême comprend un ensemble d'au moins 100 modèles avec des probabilités de sortie moyennées.

8. Procédé de la revendication 1, dans lequel le ou les biomarqueurs sont choisis dans le groupe constitué par SLC45A3, FOLH1, GHSR, JAG1, CDH11, SELE, MERTK, GABRA2, TNFRSF10B, ABCC5, LETMD1, CADM1, EMP2, ENTPD2, ABCB11, IL17RA, RNF122, ST14, SYPL1, LDLRAD3, HTR1F, EMP1, TRPV6, KCNN2, CLCN6, SLC17A3, SLC44A4, SLC22A23, C9orf91, RDH10, PNKD et TMEM229.

9. Procédé de l'une quelconque des revendications 1 à 8, dans lequel l'échantillon est un échantillon de sérum, de plasma, d'urine ou de sperme.

10. Procédé de la revendication 1, dans lequel on utilise un mélange de sondes qui lient des biomarqueurs spécifiques à un tissu, des biomarqueurs spécifiques à un cancer, des biomarqueurs spécifiques à un résultat ou une combinaison de ceux-ci, de préférence
dans lequel le biomarqueur spécifique à un tissu est un biomarqueur spécifique à la prostate, ou
dans lequel le biomarqueur spécifique à un cancer est la ghréline, ou
dans lequel le biomarqueur spécifique à un résultat est l'acide polysialique.
